# EUROPEAN PATENT SPECIFICATION

(11) **EP 2 560 985 B1**
(45) Date of publication and mention of the grant of the patent: **29.06.2016**
(21) Application number: 11718312.9
(22) Date of filing: 18.04.2011
(51) Int. Cl.: C07K 14/08, C12Q 1/70

(54) **NUCLEIC ACID SEQUENCES OF A FISH VIRUS AND THE USE THEREOF**
NUKLEINSÄURESEQUENZEN EINES FISCHVIRUS UND IHRE VERWENDUNG
SÉQUENCES D'ACIDE NUCLÉIQUE D'UN VIRUS DE POISSON ET LEUR UTILISATION

(30) Priority: 15.10.2010 NO 20101550; 21.04.2010 NO 20100571
(43) Date of publication of application: 27.02.2013
(73) Proprietor: Pharmaq AS, 0275 Oslo (NO)
(72) Inventor: HAUGLAND, Øyvind, N-0590 Oslo (NO); MIKALSEN, Aase Beathe, N-1182 Oslo (NO); EVENSEN, Øystein, N-0198 Oslo (NO); NILSEN, Pål, N-1151 Oslo (NO); LINDMO, Karine, N-0461 Oslo (NO); RODE, Marit, N-1170 Oslo (NO)
(74) Representative: Onsagers AS
(86) International application number: PCT/EP2011/056090
(87) International publication number: WO 2011/131600

(56) References cited:
- GB-A- 2 452 363
- LOVOLL MARIE ET AL: "A novel totivirus and piscine reovirus (PRV) in Atlantic salmon (Salmo salar) with cardiomyopathy syndrome (CMS).", VIROLOGY JOURNAL, vol. 7, 2010, page 309, XP000002656194, ISSN: 1743-422X -& DATABASE EMBL [Online] 22 November 2010 (2010-11-22), Lovoll et al.: XP000002656195, Database accession no. HQ401057
- HAUGLAND OYVIND ET AL: "Cardiomyopathy syndrome of atlantic salmon (Salmo salar L.) is caused by a double-stranded RNA virus of the Totiviridae family.", JOURNAL OF VIROLOGY, vol. 85, no. 11, June 2011 (2011-06), pages 5275-5286, XP009150996, ISSN: 1098-5514

## Description

The present invention relates to nucleic acid sequences originating from a virus causing cardiomyopathy syndrome (CMS) in fish and the use thereof within veterinary immunology and diagnosis. More specifically, the invention provides nucleic acid sequences coding proteins and the use thereof in inter alia recombinant vaccines, DNA vaccines and in the providing of a live recombinant vaccine. The protein coding sequences is also useful in respect of developing methods providing for immunological determination of CMS virus infection. The invention thus also relates to antibodies, and the use thereof in immunoassays.

The present invention also relates to primers useful in methods for detection of CMS virus in a test sample.

Finally, the present invention provides diagnostic kits comprising recombinant proteins, antibodies and primers, respectively.

### Background of the invention.

Cardiomyopathy syndrome (CMS) is a disease affecting primarily large Atlantic salmon in the second year in seawater close to harvest, and therefore the economic impact is significant. Affected fish may suddenly die without showing signs of disease, or may show symptoms such as abnormal swimming behaviour and anorexia. CMS is diagnosed on the basis of histopathology, showing severe inflammation and degeneration of spongious myocardium in the atrium and ventricle. A possible secondary effect of circulatory disturbance is multifocal liver necrosis which is commonly observed.

The cause of CMS was for a long time unknown. Although intracellular inclusions and virus-like particles were reported, the reports were not consistent as apparent from a review provided in Kongtorp et al. Cardiomyopathy syndrome (CMS): a literature review, National Veterinary Institute, Norway, November 2005. In 1997 Grotmol et al. reported the presence of nodavirus-like particles in endothelial cells in the heart of Atlantic salmon diagnosed as suffering from CMS. The particles had a diameter of 25 nm (Grotmol et al., 1997, Dis Aquat Org, vol 29, pp 79-84).

Experimental transmission of cardiomyopathy syndrome in Atlantic salmon, Salmo salar have been reported, cf. Bruno and Noguera, Disease of Aquatic Organism, vol. 87: 235-242, 2009 and Fritsvold et al., Disease of Aquatic Organism, vol 87: 225-234, 2009.

The infectious nature of CMS was finally demonstrated by the isolation and cultivation of a virus causing CMS in fish as reported in the Norwegian patent application NO 2008 2869. In particular, NO 2008 2869 discloses an isolated virus being deposited with the European Collection of Cell Culture (ECACC), Health Protection Agency, Porton Down, Salisbury, Wiltshire (UK), SP4 0JG UK on the 29 March 2007 under accession number 07032902.

The virus disclosed in the above mentioned Norwegian patent application may have the ability of introducing one or more symptoms selected from the group consisting of: skin haemorrhages, raised scales, exopthalamos, ascites, fibrinous casts over the liver capsule, blood or blood clots filling the pericardial cavity, ruptures in the cardiac atrial wall, dilation of the cardiac atrium, compression of the cardiac ventricle, inflammation of the spongious myocardium and the epi- and endocardium, liver lesions including multifocal to anastomosing necrosis of hepatocytes and fibrinous coating of the capsule, congestion of the spleen and/or the gills.

A further understanding of the CMS causing agent has been provided by the isolation and cultivation of the CMS virus disclosed in NO 2008 2869. The host cells for cultivation of the CMS virus disclosed in NO 2008 2869 have shown to results in low yield of the CMS virus. There is still a need for further knowledge to be able to develop efficient means for controlling the disease and to be able to develop efficient vaccines, such as recombinant vaccines. Although the virus was successfully isolated and host cells for the cultivation thereof is known from NO2008 2869, the further identification and providing of knowledge of the genome and the sequence thereof have proven difficult due to unexpected characteristics of the genome and the organisation thereof.

It is therefore an object of the present invention to provide nucleic acid sequences originating from CMS virus. Through the providing of the nucleic acid sequences of the present invention, various useful applications thereof are provided as outline below.

### Summary of the invention

The present invention thus provides an isolated nucleic acid sequence originating from CMS virus having a sequence selected from the group consisting of SEQ ID No. 1, SEQ ID No. 2, SEQ ID No. 4 and SEQ ID No. 6, and variants thereof being at least 70 % identical with any of the sequences SEQ ID No. 2, SEQ ID No. 4 and SEQ ID No. 6 based on the entire sequence length of the said isolated nucleic acid sequence.

According to one aspect the nucleic acid sequences of the invention is at least 80%, preferably 90 %, more preferably 95% identical with the nucleic acid selected from the group consisting of SEQ ID No. 2, SEQ ID No. 4 and SEQ ID No. 6 based on the entire sequence length of the said isolated nucleic acid sequence.

Another aspect of the invention relates to a vector comprising nucleic acid sequences according to the present invention, and host cells comprising said vectors.

According to another aspect of the invention, vectors are provided comprising nucleic acids selected from the group consisting of SEQ ID No. 2 and SEQ ID No. 6, and variants thereof being at least 70 % identical with any of the sequences SEQ ID No. 2, and SEQ ID No. 6 based on the entire sequence length of the said isolated nucleic acid sequence is operable linked to control sequences directing the expression of said sequences.

According to yet another aspect of the invention, DNA vaccines are provided comprising a nucleic acid sequence selected from the group consisting of SEQ ID No. 2 and SEQ ID No. 6, and variants thereof being at least 70 % identical with any of the sequences SEQ ID No. 2, and SEQ ID No. 6 based on the entire sequence length of the said isolated nucleic acid sequence.

Another aspect of the invention relates to recombinant proteins encoded by a nucleic acid sequence selected from the group consisting of SEQ ID No. 2 and SEQ ID No. 6, and variants thereof being at least 70 % identical with any of the sequences SEQ ID No. 2, and SEQ ID No. 6 based on the entire sequence length of the said isolated nucleic acid sequence. Said recombinant proteins according to the present invention has according to one aspect of the invention an amino acid sequence selected from the group consisting of SEQ ID No. 3 and SEQ ID No. 7, or a variant thereof being at least 70 % identical with any of the sequences SEQ ID No. 3, and SEQ ID No. 7 based on the entire sequence length of the said nucleic acid sequence.

The present invention also provides a recombinant vaccine comprising at least one of the recombinant proteins according to the present invention.

Furthermore, according to another aspect of the invention, an antibody is provided that recognises and binds to a recombinant protein according to the present invention. The antibody is according to one aspect a monoclonal antibody. According to another aspect, the antibody of the invention is a polyclonal antibody.

According to yet another aspect of the invention, an immunoassay is provided for the detection of CMS virus specific antibody against CMS virus in a biological sample, characterized by comprising the following steps:
a) contacting at least one of the recombinant proteins according to the present invention and a biological sample suspected to comprise antibodies against a CMS virus; and
(b) determining whether the at least one recombinant protein binds to a CMS virus specific antibody present in the biological sample wherein said binding represent an indication that the animal has been in contact with CMS virus.

According to yet another aspect of the invention an immunoassay is provided for detection of CMS virus in a biological sample, characterized by comprising the following steps:
(a) contacting one or more antibodies that specifically recognise and binds to a recombinant protein according to the invention and a biological sample suspected to comprise CMS virus; and
(b) determining whether the antibody binds to CMS virus present in the biological sample wherein said binding represent an indication that the sample contains a CMS virus.

The present invention also provides according to another aspect a primer comprising a sequence of at least about 10 nucleotides, wherein said primer hybridize to a nucleic acid sequence originating from CMS virus having a sequence selected from the group consisting of SEQ ID No. 1, SEQ ID No. 2, SEQ ID No. 4 and SEQ ID No. 6, and variants or fragments thereof being at least 70 % identical with any of the sequences SEQ ID No. 2, SEQ ID No. 4 and SEQ ID No. 6 based on the entire sequence length, of the said sequence. The primer according to the present invention may according to one aspect consist of at least 15 nucleotides, preferably at least 20 nucleotides.

According to yet another aspect of the invention, a method for the detection of CMS virus in a biological sample is provided, wherein the method comprises the following steps:
a) preparing a biological sample comprising nucleic acid sequences isolated from a biological sample suspected to comprise CMS virus for a reverse transcription reaction with a specific or random primer;
b) subjecting the mixture of a) for a polymerase chain reaction with a primer pair specific to the reverse transcribed nucleic acid sequence corresponding to the genome sequence of the virus, wherein each primer of said primer pair hybridize to a nucleic acid sequence originating from CMS virus having a sequence selected from the group consisting of SEQ ID No. 1, SEQ ID No. 2, SEQ ID No. 4 and SEQ ID No. 6, and variants thereof being at least 70 % identical with any of the sequences SEQ ID No. 2, SEQ ID No. 4 and SEQ ID No. 6 based on the entire sequence length of said sequence; and
c) determining whether the binding of the primers to nucleic acid sequences in the sample and amplification of the sequence between them have occurred indicating the presence of CMS virus in the sample tested.

According to one aspect of the above method, each primer of the primer pair is selected from a group consisting of SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17 SEQ ID No. 18, SEQ ID No. 19, SEQ ID No. 20 and SEQ ID No. 21.

Finally, the present invention relates to the use of the nucleic acid sequences according to any of the claim 1-2 for the preparation of DNA vaccine, recombinant vaccine or a live recombinant microorganism.

### Brief description of the figures

Figure 1 discloses the genomic sequence of the CMS virus corresponding to SEQ ID No. 1.
Figure 2 discloses the nucleic acid sequence of the open reading frame 1 corresponding to SEQ ID No. 2. The open reading frame 1 comprise the sequence starting at the nucleotide in position 445 and ending at the nucleotide in position 3028 of SEQ ID No. 1.
Figure 3 discloses the amino acid sequence coded by the sequence depicted in figure 2, i.e. the amino acid sequence corresponding to SEQ ID No. 3.

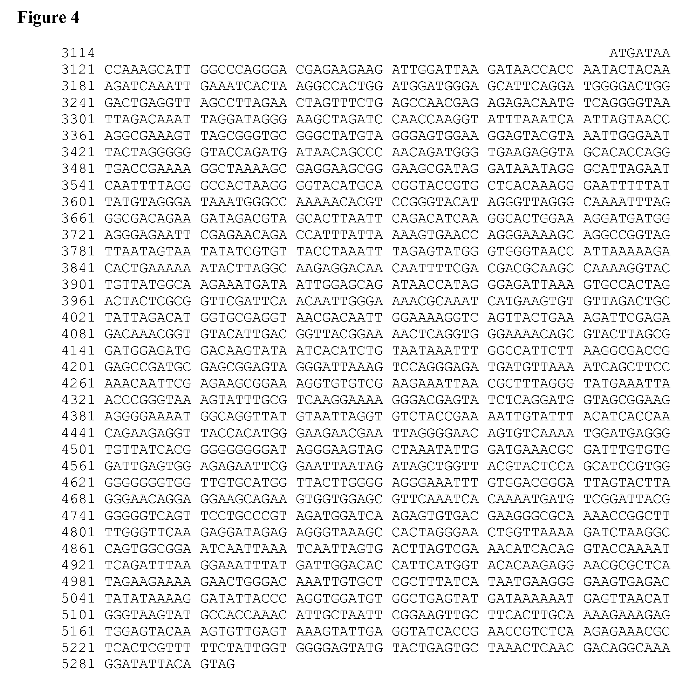

Figure 4 discloses the nucleic acid sequence of the open reading frame 2 corresponding to SEQ ID No. 4. The open reading frame 2 comprise the sequence starting at the nucleotide in position 3114 and ending at the nucleotide in position 5292 of SEQ ID No. 1.
Figure 5 discloses the amino acid sequence coded by the sequence depicted in figure 4, i.e. the amino acid sequence corresponding to SEQ ID No. 5.
Figure 6 discloses the nucleic acid sequence of the open reading frame 3 corresponding to SEQ ID No. 6. The open reading frame 3 comprise the sequence starting at the nucleotide in position 5542 and ending at the nucleotide in position 6448 of SEQ ID No. 1.
Figure 7 disclose the amino acid sequence coded by the sequence depicted in figure 6, i.e. the amino acid sequence corresponding to SEQ ID No. 7.

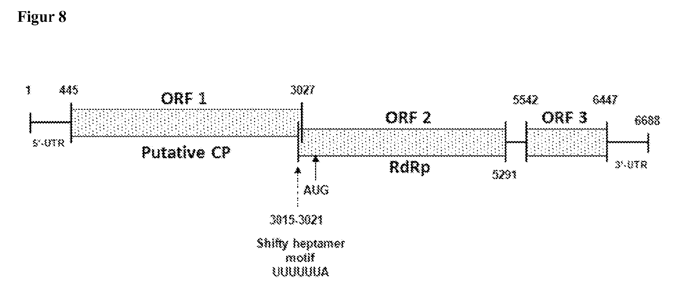

Figure 8 shows graphically the genome organization of piscine myocarditis virus (PMCV). The genome includes three large open reading frames (ORFs). The predicted ORF1 and ORF2 resemble two overlapping open reading frames. ORF2 is translated as a fusion protein with ORF1 due to ribosomal -1 frameshifting or as an individual protein (start codon indicated). ORF3 is a non-overlapping frame.

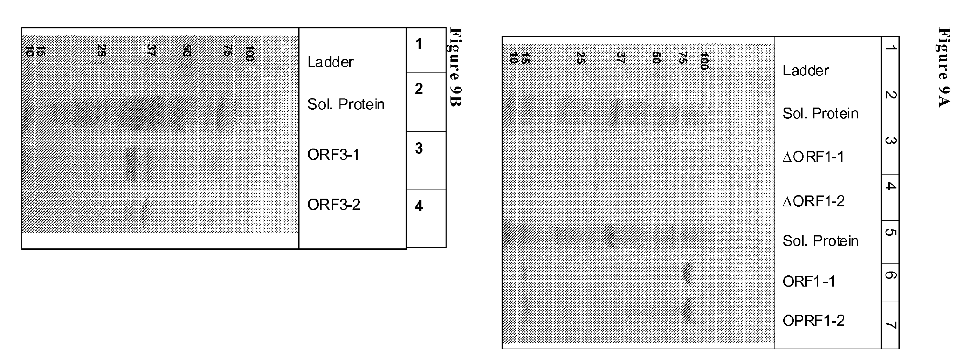

Figure 9 shows recombinant proteins coded by ORF1 and ORF3 and expressed in E.coli separated by gel electrophoresis. Figure 9A shows the expression of ORF-1 and a partial ORF 1 (nucleotide 1-852) in E.coli. The ORF1 (1-852) is expressed as a protein of approximately 30 kD (lanes 3 and 4), while the full length protein is approximately 90 kD (lanes 6 and 7). Lanes 2 and 5 are the soluble protein fractions from the fermentate. A protein size ladder is run in lane 1. Figure 9B shows the expression of ORF-3. The ORF3 is expressed as a protein of approximately 30 kD (lanes 3 and 4). Lane 2 is the soluble protein fraction. A protein size ladder is run in lane 1.
Figure 10 is a Western blot showing that an antiserum raised against recombinant ORF 1 protein produced in E.coli recognizes a band corresponding to the ORF 1 protein in heart homogenate from Atlantic salmon diagnosed with CMS (lane 2). No band of this size is recognized in heart homogenate from a healthy Atlantic salmon (lane 3). A protein size ladder is run in lane 1.
Figure 11 constitutes Western blots showing binding of ORF3 by Atlantic salmon serum from fish diagnosed with CMS (A, left panel). Control serum from healthy Atlantic salmon (B, right panel) shows no binding to ORF3. Some unspecific binding to a protein of approximately 10-15 kD is visible.
Figure 12 represents a bivariate plot showing the relationship between histoscore of spongious part of the heart ventricle and virus load (expressed as Cp values) in heart tissue for Sample 1 (n=40).

The present invention and the various embodiments thereof will now be described in detail in the following.

### Detailed description of the invention

### Definitions

The present invention relates to isolated nucleic acid sequences and variants or fragments thereof being at least 70% identical with the isolated nucleic acid sequences. The term "% identity" is to be understood to refer to the percentage of nucleotides that two or more sequences or fragments thereof contains that are the same. A specified percentage of nucleotides can be referred to as e.g. 70% identical, 80% identical, 85% identical, 90% identical, 95% identical, 99% identical or more over a specified region when compared and aligned for maximum correspondence. The skilled person will acknowledge that various means for comparing sequences are available. For example, one non-limiting example of a useful computer homology program useful for determining the percent homology between sequences includes the Basic Local Alignment Search Tool (BLAST) (Altschul et al., 1990, J. of Molec. Biol., 215:403-410, "The BLAST Algorithm; Altschul et al., 1997, Nuc. Acids Res. 25:3389-3402, , Karlin and Altschul 1990, Proc. Nat'l Acad. Sci. USA, 87:2264-68; 1993, Proc. Nat'l Acad. Sci. USA 90:5873-77).

The term "fragments or variants thereof" used in respect of the nucleic acid sequences and recombinant proteins according to the present invention is to be understood to encompass nucleic acid sequences and recombinant proteins that only differs from the isolated sequences SEQ ID No. 1-7 by way of some amino acid or nucleotide additions, deletions or alteration that have little effect, if any, on the functional activity of the claimed sequences. The skilled person will acknowledge that modifications of a protein coding nucleotide sequence may be introduced which does not alter the amino acid sequence, e.g. the substitution of a nucleotide resulting in that the triplett affected by the substitution still codes for the same amino acid. Such alterations may be introduced to adapt the nucleic acid sequence to the codons preferably used by a host cell and thus to enhance the expression of a desired recombinant protein. Furthermore, the addition of nucleic acid sequences coding polypeptides which facilitates purification may be added without affecting the activity of the resulting recombinant protein.

The skilled person will further acknowledge that also alterations of the nucleic acid sequence resulting in modifications of the amino acid sequence of the recombinant protein it codes may have little, if any, effect on e.g. the proteins ability to induce protection against CMS virus if the alteration does not have any impact on the resulting three dimensional structure of the recombinant protein. For example, a codon for the amino acid alanine, a hydrophobic amino acid, may be substituted by a codon encoding another less hydrophobic residue, such as glycin, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a recombinant protein with substantially the same functional activity as the protein having the amino acid sequences as depicted in SEQ ID No. 3, 5 and 7 and thus to be expected to constitute a biologically equivalent product. Nucleotide changes which result in alteration of the N-terminal or C-terminal portions of the protein molecule would also not be expected to alter the functional activity of the protein. Each of the proposed modifications is well within the routine skills in the art, as is determination of retention of biological activity of the encoded products. Therefore, where the terms "nucleic acid sequences of the invention" or "recombinant protein of the invention" are used in either the specification or the claims each will be understood to encompass all such modifications and variations which result in the production of a biologically equivalent protein.

Thus, the present invention therefore encompass recombinant proteins and fragments thereof which differs in respect of amino acid substitutions, addition or deletions compared with the recombinant proteins having the sequence of SEQ ID No. 3, SEQ ID No. 5, and SEQ ID No. 7, respectively, but which exert substantially the same functional activity as the proteins having the sequence of SEQ ID No. 3, SEQ ID No. 5, and SEQ ID No. 7.

The terms "antigen", immunogenic protein or peptide", "antigenic determinant" or "epitope" when used in connection with the present invention is to be understood to refer to a recombinant protein or fragment thereof according to the invention being able to induce protection against CMS in fish or to be able to bind to an antibody which recognise and bind to the CMS virus. A fragment of a recombinant protein having a sequence as depicted in SEQ ID No. 3, SEQ ID No. 5, and SEQ ID No. 7, respectively may constitute such an antigen or epitope. A non-limiting example of a fragment of the present invention is e.g. a fragment constituting for example 8-11 amino acids of the sequence SEQ ID No. 3, SEQ ID No. 5, or SEQ ID No. 7.

The term "vaccine" as used herein refers to a material that can produce an immune response that blocks the infectivity, either partially or fully, of an infectious agent, which in respect of the present invention is the cardiomyopathy syndrome virus affecting fish such as e.g. salmonids. Thus, when administering to a fish, the vaccines of the invention is immunised against the disease caused by CMS virus. The immunising component of the vaccine may be e.g. DNA as in a DNA vaccine, a recombinant protein or fragment thereof according to the present invention, or a live recombinant microorganism.

As outlined below, the present invention also provides primers (i.e. oligonucleotide sequences) useful in polymerase chain reaction techniques to be used as diagnostic tools. The term "primer" as used herein refers to an oligonucleotide either naturally occurring (e.g. as a restriction fragment) or produced synthetically which is significantly complementary to a CMS virus target sequence and thus capable of hybridizing to nucleic acid sequences of the present invention. The primer may be labelled to facilitate detection, e.g. using fluorescent labels or other label means well known to the skilled person.

### Detailed description of the embodiments of the invention

### Nucleic acid sequences

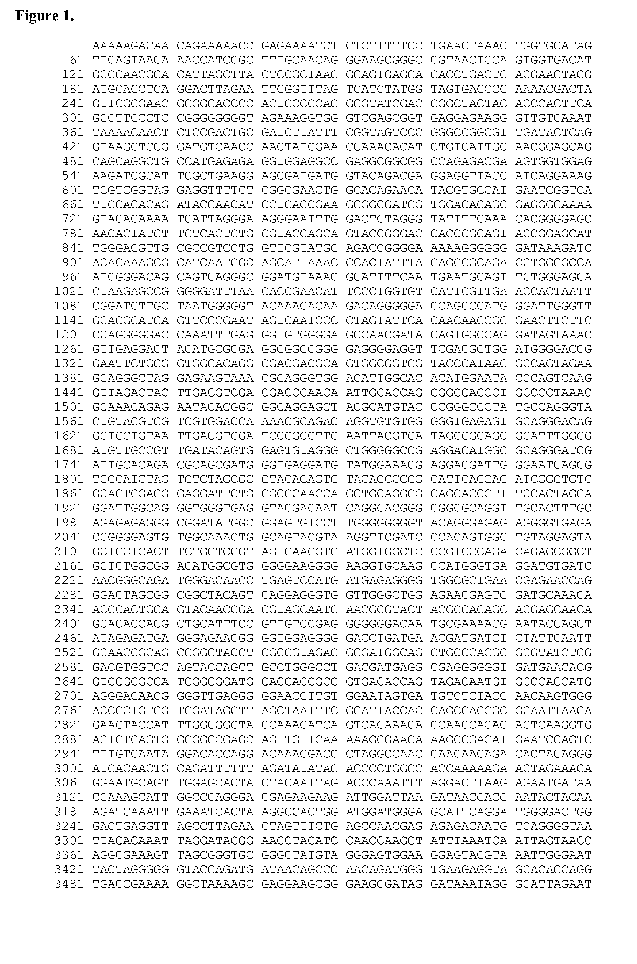

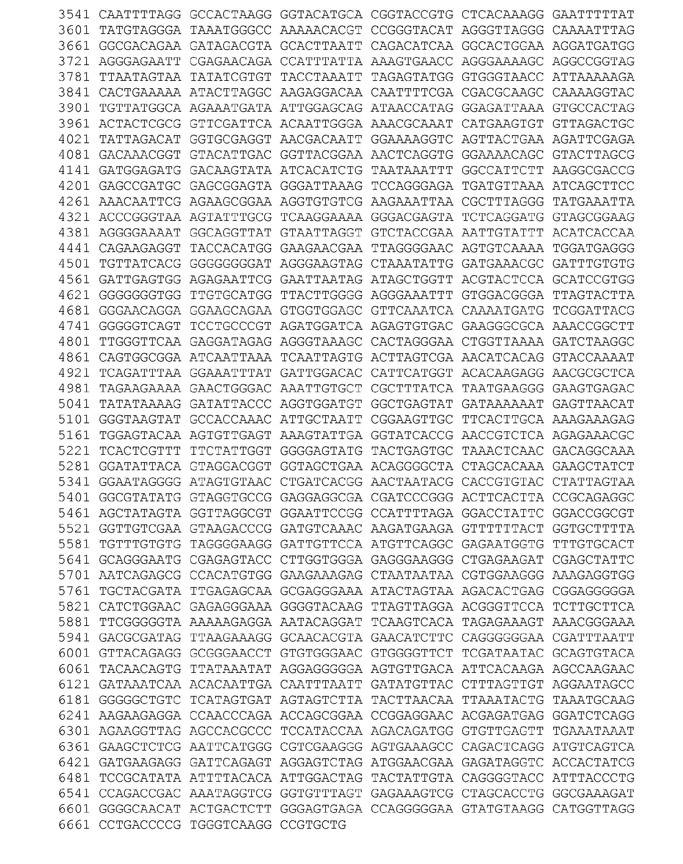

The present inventors have provided the genomic sequence of the CMS virus. GF-1 cells were infected with homogenate obtained from salmon suspected of being infected with CMS virus. Culture supernatant was harvested, pelletted by centrifugation and subjected to DNase and RNAse treatment. RNA was then isolated and cDNA synthesised using random primers. Thereafter, double stranded DNA was provided and amplified by PCR using random primers, and the obtained products were sequenced and analysed to obtain part of the genomic sequence according to the present invention depicted in figure 1 and SEQ ID No. 1. The sequence of the rest of the genome was obtained by PCR with specific primers and 3' and 5' RACE (Rapid Amplification of cDNA Ends).

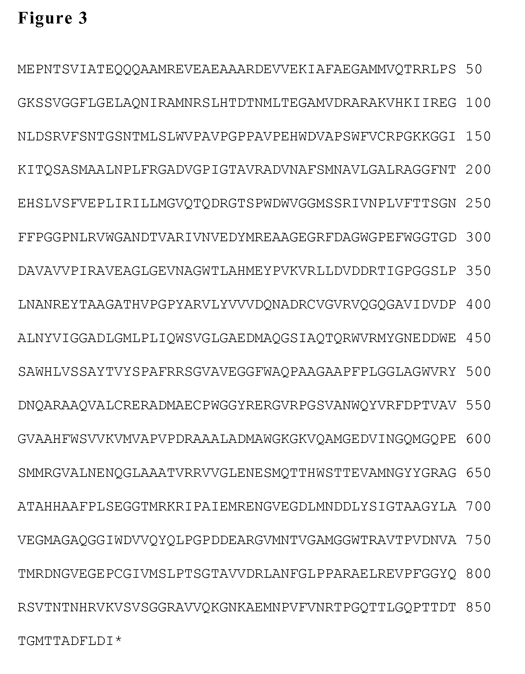

The further analysis of SEQ ID No. 1 by the present inventors revealed three open reading frames which are believed to include the coding region for three viral proteins. Sequence comparisons with other known viral sequences has revealed that the open reading frame 1 (SEQ ID No. 2) according to the present invention shows very weak homology to other viral sequences. Furthermore, the analysis of the open reading frame 1 (SEQ ID No. 2) indicates that it comprise a nucleic acid sequence coding a coat protein. The amino acid sequence of the open reading frame 1 is shown in figure 3 and SEQ ID No. 3. Said sequence show only very weak homology with other known proteins. (Poilos et al., 2006, Jour. General Virology, 87, 987-996, Tang et al. 2008, PNAS,105:45, 17526- 17531).

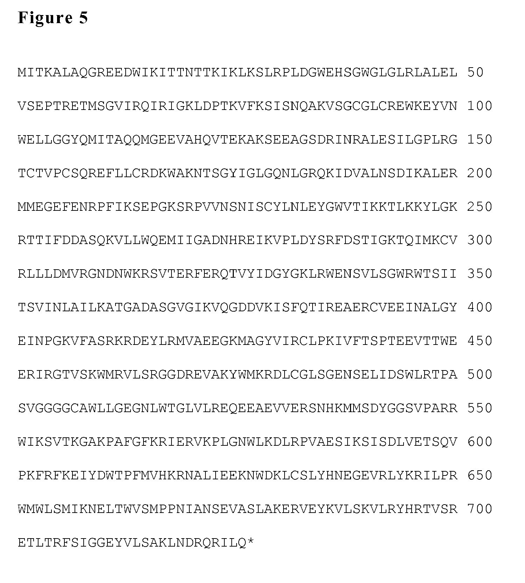

A further isolated nucleic acid sequence of the invention (open reading frame 2, SEQ ID No. 4) comprises a sequence coding an RNA dependent RNA polymerase and the amino acid sequence thereof is depicted in figure 5 and SEQ ID No. 5. Comparison with other known viral polymerase coding sequences shows that this sequence is weakly identical with a totivirus polymerase, in particular a *Giardia lamblia virus* which belongs to Totiviridae when a homology search is conducted with the BLAST program (Accession no. AAB01579).

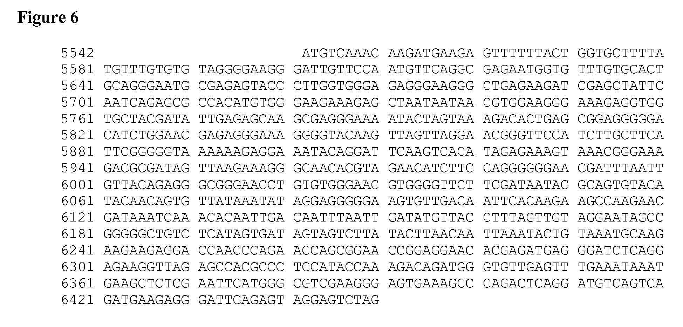

The third open reading frame (SEQ ID No. 6) of the invention comprises a sequence which is also believed to include the coding sequence of a coat protein, the amino acid sequence thereof being depicted in figure 6/SEQ ID No. 6. Surprisingly, said sequence show only very weak homology with other viral coat proteins, and no other members of the Totiviridae family comprise this particular open reading frame. (Poilos et al., 2006, Jour. General Virology, 87, 987-996).

With the providing of the above sequences, further progress in respect of controlling and preventing cardiomyopathy syndrome in fish is achieved. The sequences of the open reading frames 1 and/or 3 (SEQ ID No. 2 and 6) may form the basis for the development of recombinant proteins to be used in a vaccine, or the development of DNA vaccines or a live recombinant microorganism.

Recombinant proteins being coded for by sequences based on the open reading frames 1 and 3 may also form the basis for the development of diagnostic tools, i.e. through the production of antibodies and the development of immunoassays for the detection of the presence of CMS virus in biological samples.

Finally, the sequences provided may form the basis for development of diagnostic tools wherein the nucleic acid sequences or fragments thereof is used to detect the presence of CMS virus in fish.

The various embodiments of the invention being the result of the providing of the nucleic acid sequences of the invention are outlined further below. The skilled person will acknowledge that said embodiments should not be understood to involve a limitation of the scope of the claimed nucleic acid sequences. Other uses of the claimed nucleic acid sequences are also understood to be covered by the scope of the present invention.

### Recombinant proteins

According to one aspect of the invention, the invention provides recombinant cardiomyopathy virus proteins.

The skilled person is well familiar with the various available biotechnological techniques providing for the expression of isolated nucleic acid sequences for the preparation of recombinant proteins by heterologous expression in various host cell systems using commonly available genetic engineering techniques and recombinant DNA expression systems. Various protocols for the expression of recombinant protein are known, cf. e.g. "Recombinant Gene Expression Protocols, in Methods in Molecular Biology, 1997, Ed. Rocky S Tuan, Human Press (ISSN 1064-3745) or Sambrook et al., Molecular Cloning: A laboratory Manual (third edition), 2001, CSHL Press, (ISBN 978-087969577-4). For example, the nucleic acid sequences of the open reading frames 1 and 3 or fragments or variants thereof may be inserted in suitable expression vectors comprising all the necessary transcriptional and translational regulatory sequences specifically adapted for directing the expression of the desired protein coding nucleic acid sequence in a suitable host cell. Suitable expression vectors are e.g. plasmids, cosmids, viruses or artificial yeast chromosomes (YAC's). A non-limiting list of examples of suitable expression vector systems is e.g. pET-type of vectors (available from Invitrogen), pcDNA3.1 vectors (available from Invitrogen), or the pBR-type, pUC-type or pGEM-type of expression vectors. The obtained expression vector including the open reading frames (SEQ ID No. 2, or SEQ ID No. 6) or a fragment thereof is then introduced in suitable host cells for the production of the desired recombinant protein.

According to one embodiment of the invention, a vector is provided comprising a sequence selected from the group consisting of SEQ ID No. 2 and SEQ ID No. 6, and variants or fragments thereof being at least 70 % identical with any of the sequences SEQ ID No. 2, and SEQ ID No. 6 operatively linked to control sequences directing the expression of said sequences. The term "operatively linked", means that the desired sequence coding for a recombinant protein is linked to all the necessary transcriptional and translational regulatory sequences specifically adapted for directing the expression of the desired protein coding nucleic acid sequence in a suitable host cell.

It should be understood that various modifications may be introduced in the nucleic acid sequences of the present invention utilising techniques well known to the skilled person for example to facilitate expression. By the use of site directed mutagenesis or synthesis, modification may be introduced to adapt the coding sequence to the desired host used to express the sequence and thus produce the recombinant protein. The skilled person is well aware of the fact of the presence of host specific codons, and that the adaption of a heterologous nucleic acid sequence to the host specific codons increase the expression efficiency. Other modification may also be introduced, e.g. to facilitate isolation and purification, i.e. by adding a sequence coding for a peptide or protein useful for such purposes. Also, nucleic acid sequences coding signal peptide providing for secretion of the desired recombinant protein from the host cell may also be linked to the nucleic acid sequences of the present invention. In case the recombinant protein is expressed in a live recombinant microorganism (see below), the nucleic acid sequence may be modified to enable the anchorage of the recombinant protein in the membrane or cell wall of the microorganism resulting in the presentation of the recombinant protein on the surface of the live recombinant microorganism. According to such an embodiment of the invention, the nucleic acid sequence of the present invention is modified by adding a nucleic acid sequence coding a protein or peptide resulting in the anchorage of the resulting recombinant protein in the membrane or cell wall of the live recombinant microorganism.

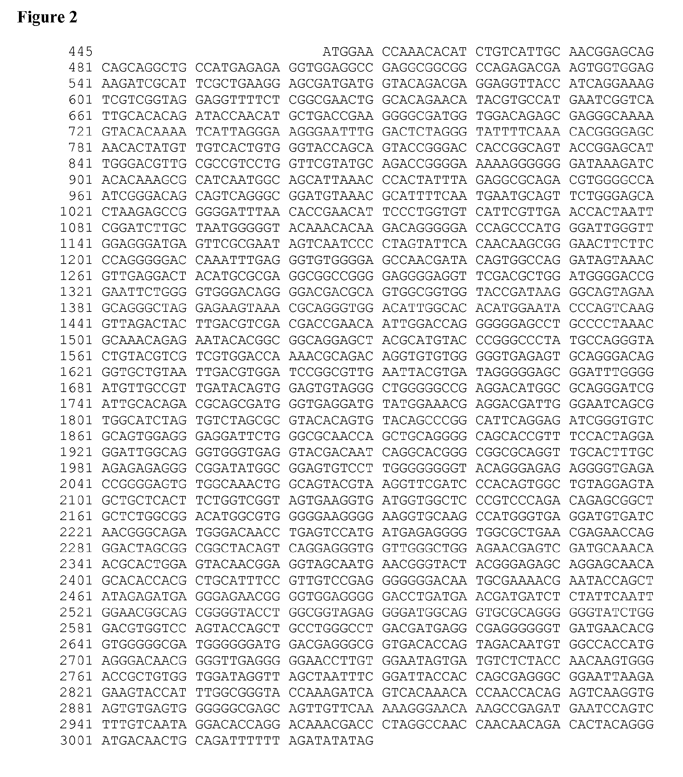

The present invention thus provides vectors comprising the nucleic sequence as depicted in figure 2 and/or 6 (SEQ ID No. 2 and SEQ ID No. 6) or variants or fragments thereof. The present invention also provides host cells comprising the vectors of the invention.

Various commercially available host cells specifically adapted for the production of recombinant proteins may be used, both prokaryotic host cells and eukaryotic host cells. Non-limiting examples of suitable prokaryote host cells is e.g. *Escherichia Coli, Bacillus substilis*, *Lactobacilus sp, Caulobacter cresentus*, *Yersinia ruckeri*, *or Vibrio anguillarum*, Non-limiting examples of suitable eukaryote host cells is e.g. yeast such as *Saccharomyces cerevisiae or Chinese hamster ovary cells.* Also viruses may be used for the production recombinant expression of a desired protein, cf. e.g. alphavirus, adenovirus or baculovirus vector systems (Bac-to-Bac® Baculovirus Expression Systems, Invitrogen, PichiaPink™ Yeast Expression System, Invitrogen).

According to one embodiment of the invention, a pET vector available from Invitrogen and specifically adapted for the expression of recombinant proteins in *Escherichia Coli* is used.

Recombinant protein may also be provided by using cell free expression systems. Recombinant proteins being the results of expression of the open reading frames 1 or 3 (SEQ ID No. 2 or 6) or fragments or combinations thereof may be used in a vaccine to obtain protection against further CMV virus infections.

### Recombinant vaccine composition

Recombinant proteins prepared by heterologous expression of the nucleic acid sequences according to the present invention may be used as immunogenic component(s) of a recombinant vaccine composition.

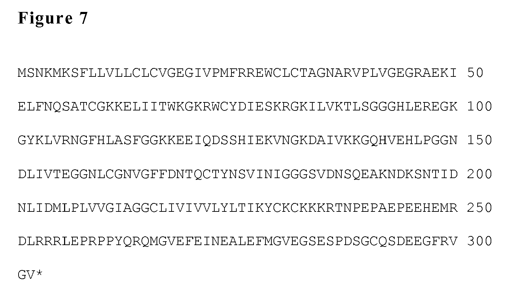

The present invention therefore provides a vaccine composition comprising at least one recombinant protein having an amino acid sequence as depicted in figure 3 or 7 (SEQ ID No. 3 or SEQ ID No. 7), or fragments or variants thereof.

According to one aspect of the invention, the at least one recombinant protein used in a vaccine consist of a fragment of the coating protein coded for by the sequences of the open reading frames 1 or 3 that induce an immune response in the individual being administered the recombinant vaccine of the invention. Such fragments may constitute an epitope of the coating protein coded for by the open reading frames 1 or 3, also known as an *antigenic determinant.* The skilled person is well familiar with the fact that the coat proteins of a virus may comprise one or more epitopes that constitutes the part of the coat protein that is recognized by the immune system and thus result in the development of protection against further infection. The epitopes may be found as linear, continuous sequence of amino acids, such as e.g. 8-11 amino acids, within a coat protein or another surface structure of a pathogen organism. Such an epitope is commonly named a linear epitope. An epitope of a coat protein may also be the result of a three dimensional structure of the coat protein, e.g. formed by the assembly of amino acids of the sequence that are not sequential. Such epitopes are commonly named discontinuous epitopes, and are often larger than a linear epitope.

Linear and discontinuous epitopes comprised in the open reading frame 1 and 3 of the invention may be determined by the use of epitope mapping technology available to the skilled person, c.f. Methods in Molecular Biology: Epitope Mapping Protocols, 1996, vol. 66, Ed. Glenn E Morris, Human Press (ISBN 978-0-89603-375-7). It is to be understood that vaccines comprising recombinant proteins according to the present invention which constitute protective epitopes of the coat proteins of CMS virus prepared by expression of fragments of the open reading frames 1 or 3, or variants or fragments thereof, is covered by scope of the present invention. More specifically, epitopes which are recognized by neutralizing antibodies will be of particular interest.

Epitopes that are found in those parts of the recombinant proteins according to the present invention that are particular specific for the CMS virus are preferred.

The recombinant vaccine composition according to the invention comprising recombinant proteins or fragments thereof as outlined above may further comprise an adjuvant. Examples of adjuvants frequently used in fish and shellfish farming are muramyldipeptides, lipopolysaccharides, several glucans and glycans, mineral oil, Montanide™ and Carbopol^{®}. An extensive overview of adjuvants suitable for fish and shellfish vaccines is given in the review paper by Jan Raa, 1996, Reviews in Fisheries Science 4(3): 229-228.

The vaccine of the invention may further comprise a suitable pharmaceutical carrier. In a currently preferred embodiment the vaccine is formulated as an emulsion of water in oil. The vaccine may also comprise a so-called "vehicle". A vehicle is a device to which the antigen adheres, without being covalently bound to it. Such vehicles are i.a. biodegradable nano/micro-particles or -capsules of PLGA (poly-lactide-*co*-glycolic acid), alginate or chitosan, liposomes, niosomes, micelles, multiple emulsions and macrosols, all known in the art. A special form of such a vehicle, in which the antigen is partially embedded in the vehicle, is the so-called ISCOM (European patents EP 109.942, EP 180.564 and EP 242.380)

In addition, the vaccine may comprise one or more suitable surface-active compounds or emulsifiers, e.g. Cremophore®, Tween® and Span®. Also adjuvants such as interleukin, CpG and glycoproteins may be used.

It is to be understood that the vaccine may further be in a formulation comprising an antigen from a bacterial source, an antigenic material obtained from a viral source other than the fish virus as defined above, an antigenic material obtained from a parasitical source, and/or an antigenic material obtained from a fungal source. Polyvalent vaccines containing antigens from typical fish pathogens other than CMS virus are well known in the art and are already commercially available. In addition, representative isolates of relevant fish pathogens are available from various sources.

In particular embodiments of the invention said antigen from a bacterial source is selected from the group consisting of: live, attenuated or killed bacteria of the species *Piscirickettsias sp. Aeromonas sp., Vibrio sp., Listonella sp., Moritella viscosa, Photobacterium damsela, Flavobacterium sp., Yersinia sp., Renibacterium sp., Streptococcus sp., Lactococcus sp., Leuconostoc sp., Bifidobacterium sp., Pediococcus sp., Brevibacterium sp., Edwarsiella sp., Francisella sp., Pseardomonas sp., Cytophaga sp., Nocardia sp., Mycobacerium sp.,* parts or subunits of these bacteria, and any combination hereof.

Isolates of such bacteria are available, e.g. from LGC Promochem/American Type Culture Collection ATCC repository and distribution center (ATCC) including strains of *A*. *salmonicida* (ATCC 33658), *V. salmonicida* (ATCC 43839), V. *anguillarum* serotype O1(ATCC 43305) and O2(ATCC 19264), and *Moritella viscosa* (ATCC BAA-105). In addition, cultures of *Piscirickettsias salmonis* have been deposited in the European Collection of Cell Culture (ECACC), Health Protection Agency, Porton Down, Salisbury, Wiltshire (UK), SP4 0JG UK on the 9 June 2006 under the following accession numbers: 06050901, 06050902, 06050903 and 07032110.

Other specific embodiments pertain to a vaccine, wherein said antigenic material obtained from a viral source other than the fish virus as defined above is from a virus selected from the group consisting of: Viral Hemorrhagic Septicemia Virus (VHSV), Viral Hemorrhagic Septicemia Virus (VHSV), Infectious Hematopoietic Necrosis virus (IHNV), Infectious Pancreatic Necrosis Virus (IPNV), Spring Viremia of Carp (SVC), Channel Catfish Virus (CCV), Infectious Salmon Anaemia virus (ISAV), pancreatic disease virus (SPDV), Iridovirus, and heart and skeletal muscle inflammation virus (HSMIV), parts or subunits of any one of these viruses, and combinations hereof. Representative species of such viruses are available to the skilled artisan, for instance from the following deposits: infectious pancreatic necrosis virus (IPNV, ATCC VR-1318, country of origin: unknown), Viral Hemorrhagic Septicemia Virus (VHSV, ATCC VR_1389, country of origin: Denmark); Infectious Hematopoietic Necrosis virus (IHNV, ATCC VR-1392, country of origin: USA)); Pancreatic Necrosis Virus; Spring Viremia of Carp (SVC, ATCC VR-1390, country of origin: Denmark); Channel Catfish Virus (CCV) (ATCC VR-665, country of origin: USA); Infectious Salmon Anaemia (ISA) virus (ATCC VR-1554, country of origin: Canada).

Patent deposits have previously been made by the present applicant of the following viral species: Heart and Skeletal Muscle Infection Virus (HSMIV, patent deposit nr ECACC 04050401, country of origin: Norway).

In more specific embodiments, said antigenic material obtained from a viral source other than the fish virus as defined above is from the group consisting of: Glycoprotein of Viral Hemorrhagic Septicemia Virus (VHSV), nucleoprotein of Viral Hemorrhagic Septicemia Virus (VHSV), glycoprotein of Infectious Hematopoietic Necrosis virus (IHNV), structural proteins of Infectious Pancreatic Necrosis Virus (IPNV), G protein of Spring Viremia of Carp (SVC), and a membrane-associated protein, tegumin or capsid protein or glycoprotein, of Channel Catfish Virus (CCV), antigenic fragments of any of one of these proteins and combinations hereof.

In other embodiments said antigenic material from a parasitic source is from a source selected from the group consisting of *Lepeophtheirus Sp., Caligus Sp.,* and *Ichthyophthirius Sp,* parts of any one of these parasites, and combinations hereof.

In yet other embodiments said antigenic material is from a fungal source selected from the group consisting of *Saprolegnia Sp., Branchiomyces sanguinis, Branchiomyces demigrans* and *Icthyophonus hoferi*.

The vaccine according to the invention may in particular be formulated for administration to a fin fish. More specifically the vaccine may be (formulated) for administration to a telostei. The teleostei include, but are not limited to salmonids, basses, breams, cods, snappers, flatfish, catfish, yellowtails and tilapias.

In a presently preferred embodiment the vaccine is formulated for administration to Atlantic salmon (*Salmo Salar* L.), Rainbow trout (Oncorhynchus mykiss) and/or Coho salmon (Oncorhychus kisutch).

In further embodiments of the invention the vaccine is formulated for administration by a route selected from the group consisting of: bath, immersion, intraperitoneal injection, intramuscular injection and oral administration. Optionally, the vaccine would be administered to young fish in the fresh-water stage.

### Live recombinant microorganism

According to another embodiment of the invention, the sequences of the open reading frames 1 and/or 3 may also be used to develop a live recombinant microorganism to be used in a vaccine against CMS virus infection. A live recombinant organism function as a carrier for genetic information according to the present invention by the insertion of the open reading frames 1 or 3 or variants or fragments thereof. Said open reading frames or variants or fragments thereof may be inserted in the microorganism in a way making the microorganism capable of expressing said nucleic acid sequences. The nucleic acid sequences or variants or fragments thereof according to the invention may be inserted in the genome of the microorganism or by the introduction of a vector comprising the desired nucleic acid sequences. The live recombinant microorganism may thus be a carrier of nucleic acid sequences coding for the recombinant proteins or variants or fragments thereof according to the present invention. As mentioned above, the live recombinant organism may e.g. carry one or more recombinant proteins according to the present invention on the surface, e.g. wherein said recombinant proteins are anchoraged in the membrane or the cell wall of the live recombinant organism.

The live recombinant microorganism may be e.g. a virus, a bacteria or a parasite. The live recombinant microorganism may function in the same way as a live fish vaccine, i.e. the microorganism will replicate in the target fish and result in an immunological response against the recombinant proteins coded by the nucleic acids of the invention.

The live recombinant microorganism may have the advantage of being efficiently cultured and thus being optimised for desired fermentation processes and high yield production of vaccine.

The skilled person will be able, by the use of common biotechnological techniques for cloning and constructions of recombinant organisms, to construct a live recombinant microorganism according to the present invention.

The live recombinant microorganism may also be the carrier of nucleic acid coding for other fish pathogens thus providing for protection against a variety of pathogens. The live recombinant organism may thus include nucleic acids coding e.g. coating proteins or fragments thereof of other fish viruses, such as e.g. SAV, IPNV, IHNV or HSMIV.

According to one embodiment of the invention, the live recombinant microorganism is a virus able to replicate in fish, preferably salmonid fish. For example, IHNV may be used as live recombinant microorganism, c.f. WO 03/097090 reporting the use of a modified novirhabdovirus to obtain vaccines. The use of live recombinant microorganism as carrier for nucleic acids coding antigens is also suggested for other fish pathogens, e.g. in WO 2007/031572.

A vaccine composition comprising a live recombinant microorganism may further as for the recombinant vaccine described above comprise adjuvants, carrier, surface-active compounds or emulsifiers as those described above. Furthermore, the advantage of combining different vaccine components is equally applicable to the live recombinant vaccine according to the present invention as to recombinant vaccine composition as disclosed above.

### DNA vaccine

According to one aspect of the invention, a DNA vaccine is provided comprising nucleic acids according to the present invention.

DNA vaccines is based on the administration of nucleic acids sequence(s) coding an antigen to a patient, and wherein the expression of said nucleic acid(s) results in an immune response in said patient. For the purpose of the present invention, the patient is a fish. A DNA vaccine may consist of a naked plasmid DNA comprising a nucleic acid sequence coding the desired antigen of a pathogen operably linked to suitable promoter sequences and other suitable control sequences enabling the expression of said nucleic acid sequence by the cells of the patient after administration thereof. A DNA vaccine may also be carried in a live host microorganism being involved in the delivering the genetic material to the patient.

Various DNA vaccines to combat fish pathogens are known. Heppell et al. reports of the protection against live virus after injection with a DNA vaccine comprising viral haemorrhagic septicaemia virus G and N genes cloned into an expression plasmid and injected into rainbow trout (Fish and Shellfish Immunology, 1998, vol 8, issue 4, pp 271-286). Lorenzen and LaPatra describes a DNA vaccine against a fish rabdhovirus in "DNA vaccines for aquacultured fish", Rev. sci.tech. Off int. Epiz., 2005 (1), 201-213. A DNA vaccine resulting in protective effects is further more reported by Mikalsen et al., 2005, "Protective effects of a DNA vaccine expressing the infectious salmon anaemia virus hemagglutinin esterase in Atlantic salmon" , Vaccine, 23:30, pp 4895-4905.

In EP 1 818 406 A1, a DNA expression system that may be used to produce a DNA vaccine that prevents infectious disease in aquatic animals is described, wherein the expression is directed by a cytomegalovirus promoter.

WO 2007/31572 discloses inter alia a DNA vaccine useful to combat SAV infections in salmonid fish.

WO 2009/002376 discloses a DNA vaccine useful to combat viral infection in carp (Spring viremia of carp virus).

EP 2 011 876 regards the development of a DNA fish vaccine useful to combat KOI Herpes disease that affects carps.

Based on the prior art and the common general knowledge referred to above, the skilled person is able to construct a DNA vaccine comprising the open reading frame 1 or 3 of the present invention or fragments or variants thereof utilizing methods commonly used in the field of genetic engineering.

DNA vaccines consisting of naked plasmid comprising the open reading frames 1 or 3 or fragments or variant thereof may be administered to fish by intramuscular injection or particle mediated deliver by gene gun. The latter involves the coating of small gold particles with the DNA vaccine, and the administration is then effected by air-pressured mediated intradermal delivery. Intramuscular injection of a DNA vaccine according to the present invention is preferred as it is more cost effective than the gene gun method, cf. Lorenz and LaPatra, *supra*. Methods for the administration of DNA vaccines to fish is furthermore disclosed in "Intramuscular Injection of DNA vaccines in Fish" by Heppel and Davis in "DNA Vaccines: Methods and Protocols", Methods of Molecular Medicine, 2000, vol. 29 (ISBN 978-0-89603-508-5).

### Antibodies and immunoassays

The recombinant proteins according to the present invention may be used to obtain antibodies capable of binding a CMS virus and thus being useful in immunological methods designed for the determination of CMS virus in biological samples.

Another aspect of the invention thus relates to an antiserum or an isolated antibody or antibodies (monoclonal or polyclonal) which selectively binds to a CMS virus or to a component or part of said virus. Antiserum is conventionally obtained for instance by immunising a laboratory animal with the appropriate antigenic determinant. Once the concentration of antibodies in serum from the animal reaches a desired level, the animal is bled. The serum thus obtained should contain antibodies produced in response to the immunogenic stimulus.

Likewise, techniques for the preparation of antibodies are known to the skilled person. The techniques include the traditional hybridoma technology and alternative techniques such as mRNA display, ribosome display, phage display and covalent display.

In some embodiments of the invention the isolated antibody comprises a marker, e.g. a radiolabel, a fluorescent tag, a chemiluminescent label or an enzyme. The antibody may be a polyclonal or monoclonal antibody. Protocols for the labelling of antibodies by coupling of the various markers mentioned above are well known to the skilled person.

For sufficient protection and control of the disease caused by CMS virus, means for efficient diagnosis of CMS virus is needed. The present invention therefore provides immunoassays, useful for the diagnosis of CMS virus in fish. An immunoassay within the meaning of the present invention is a biochemical test that measures the presence and/or concentration of the CMS virus in a biological sample, such as e.g. serum. An immunoassay thus utilise the specific binding of an antibody to its antigen. Both the presence of antigens (e.g. present on the CMS virus) and the presence of antibodies against the CMS virus may be measured in the biological sample.

The present invention further provides an immunoassay for detection of CMS virus in a biological sample, comprising (a) contacting the recombinant proteins according to the present invention and a biological sample suspected to comprise antibodies against a CMS virus; and (b) determining whether the one or more antibodies binds to a CMS virus present in the biological sample wherein said binding represent an indication that the sample contains a CMS virus.

According to another aspect of the invention, an immunoassay for detection of CMS virus in a biological sample is provided, comprising (a) contacting one or more antibodies that specifically recognise and binds to a recombinant protein according to the invention and a biological sample suspected to comprise CMS virus; and (b) determining whether the antibody binds to CMS virus present in the biological sample wherein said binding represent an indication that the sample contains a CMS virus.

The complex formed in the immunoassays according to the present invention, e.g. the binding of an antibody to a CMV virus present in a biological sample or the binding of an antibody present in a biological sample to a recombinant protein or fragment thereof according to the present invention, may be determined by means of e.g. densitometry, fluorimetry or colorimetry or the like.

The immunoassays of the invention as described above may be a standard Enzyme-linked immunosorbent assay (ELISA). The skilled person will be able to provide ELISA protocols using antibodies developed against the recombinant proteins according to the present invention, or the recombinant proteins. Reference is in this respect made to the ELISA Guidebook, Methods in Molecular biology, vol. 149, ed. By John R Crowther, Humana Press, 2001.

The present invention also provides a diagnostic kit for carrying out the immunoassays according to the present invention. According to one embodiment, the kit according to the invention comprise antibodies recognizing a recombinant protein according to the invention and usual means for carrying out an immunoassay, such as e.g. wells of an ELISA plate, reagent to produce a colour reaction upon the binding of the antibodies to a CMS virus in a biological sample to be tested. According to another embodiment, a kit is provided comprising one or more recombinant proteins according to the invention and usual means for carrying out an immunoassay, such as e.g. wells of an ELISA plate, reagent to produce a colour reaction upon the binding of said recombinant proteins to antibodies in a biological sample to be tested.

### Primer sequences and method of detection of CMS virus infection

The nucleic acid sequences of the present invention may furthermore be used to detect the presence of CMS virus in fish. The present invention therefore also relates to a diagnostic method wherein primers specifically hybridize to a nucleic acid in a test sample that is similar to the nucleic acid sequences of the invention. Such tests may take the form of a PCR test or a hybridization test without amplification.

The detection of nucleic acids present in a biological sample is widely applied in both human and veterinary diagnosis, wherein nucleic acids from e.g. pathogens present in biological samples are isolated and hybridized to one or more primers. The one or more primers to be used in hybridization based detection methods is constructed so that they are able to specifically bind to a nucleic acid sequence if present in the sample to be tested. The primers may be linked to a signalling unit, such as e.g. a radiolabel, a luminescent molecule or a fluorescent molecule enabling the visualisation of the binding of the primers to a target sequence.

A primer as used herein refers to a nucleic acid sequence, which can be used as a probe in a method of detection of the presence of CMS virus in fish. The primers according to the present invention consist of at least 10, preferably at least 15, and more preferably at least 20 nucleotides that are able to hybridize to nucleic acid sequences isolated from a CMS virus.

The primer according to the present invention is able to hybridized to another nucleic acid molecule, such as genomic RNA originating from CMS virus or cDNA prepared from said genomic RNA, under appropriate conditions of temperature and solution ionic strength, cf. e.g. Sambrook et al., Molecular Cloning: A laboratory Manual (third edition), 2001, CSHL Press, (ISBN 978-087969577-4). The condition of temperature and ionic strength determine what the skilled person will recognise as the "stringency" of the hybridization. The suitable stringency for hybridisation of a primer to target nucleic acids depends on inter alia the length of the primer and the degree of complementation, variables well known to the skilled person. A minimum length of a hybridisable primer is usually at least about 10 nucleotides, preferably about 15 nucleotides and more preferably about 20 nucleotides.

Various protocols for detection of nucleic acid sequences by PCR are available to the skilled person; cf. e.g. Methods in Molecular Biology, vol. 226, PCR protocols, second ed., Ed. Barlett and Stirling, Humana Press, 2003. The skilled person will furthermore, with the knowledge of the genomic CMS virus sequence depicted in figure 1 (SEQ ID No. 1) be able to provide suitable primers for the purpose of using said primers in a method for detection of the presence of CMS virus according to the present invention. Reference is in this respect made to the textbook PCR Primer Design, vol. 402 of the series Methods in Molecular Biology, Ed. A. Yuryev, 2007, Humana Press.

According to one embodiment of the invention, the following primers are provided:

| |
|---|
| 5'-TCCAGTGCCTTGATGTCTG-'3 (SEQ ID NO. 8) |
| 5'-CATCTCCATCCGCTAAGTACG-'3 (SEQ ID NO. 9) |
| 5'-TGCCTGTCGTTGAGTTTAGC-'3 (SEQ ID NO. 10) |
| 5'-CCCGAATGAAGCAAGATGG-'3 (SEQ ID NO.11) |
| 5'-GAAAGCCCAGACTCAGGATG-'3 (SEQ ID NO. 12) |
| 5'-ACACCAGGTGACCGAAAAG-'3 (SEQ ID NO. 13) |
| 5'-ACATGGTGCGAGGTAACGAC-'3 (SEQ ID NO. 14) |
| 5'-AGTTCCTGCCCGTAGATGG-'3 (SEQ ID NO. 15) |
| 5'-GGCGAGAATGGTGTTTGTG-'3 (SEQ ID NO. 16) |
| 5'-CCTGGTCTCACTCCCAAGAG-'3 (SEQ ID NO. 17) |
| 5'- AGGGAACAGGAGGAAGCAGAA -'3 (SEQ ID.NO 18) |
| 5'- CGTAATCCGACATCATTTTGTG -'3 (SEQ ID NO. 19) |
| 5'- GGAAGCAGAAGTGGTGGAGCGT-'3 (SEQ ID NO. 20) |
| 5'-CCGGTTTTGCGCCCTTCGTC-'3 (SEQ ID NO. 21) |

### Experiments

### Example 1. Identification of virus sequences.

Although isolation of the virus causing CMS was reported in patent application NO2008 2869, identification of the genome of this virus has proven difficult. The virus is cultivatable in GF-1 cells, but the output of virus is very low in this system. Many different methods for cloning sequences from unknown viral genomes have been tested, but none with success.

Previous studies indicate that CMS is a chronic disease developing over several months prior to the terminal clinical phase. Consistent histopathological changes are observed only in the heart, and sometimes histopathological changes are observed in the liver as well. Nothing is known about the distribution of virus at the different stages of the disease. It is often difficult to give salmon a clear CMS diagnosis, as many other diseases give the same clinical signs i.e. pancreas disease and heart and skeletal muscle inflammation (Kongtorp RT, Halse M, Taksdal T, Falk K, J Fish Dis. 2006 Apr;29(4):233-44.

Gf-1 cells were infected with homogenate made from Atlantic salmon originating from an aquaculture site experiencing disease problems compatible with CMS. After 14 days, cell cultures started to show mild cpe (cytopathogene effect), and this developed into a stronger cpe after 21 days. The cpe was not possible to reproduce in more than three passages, and became weaker at each passage.

50 ml of supernatant from infected cultures were collected, passed through a 0.22 µm filter and centrifuged at 100 000 x g for three hours to collect the virus as a pellet. After removal of almost all supernatant from the resulting virus pellet, the pellet was resuspended, and the volume adjusted with fresh cell culture medium resulting in 500µl virus suspension. This was digested with 250U DNAse I and 5µg RNAse A in RDD-buffer (included with Qiagen DNAse I) for 1h at 37°C.

RNA was prepared from the sample using QIAamp viral kit (Qiagen) according to the manufacturers instructions. Single stranded cDNA was synthesized using SuperScript®III reverse transcriptase kit (Invitrogen) and random primers with a fixed 5' overhang (FR26RVN primer 5' GCC GGA GCT CTG CAG ATA TCN NNN NN 3'; Djikeng et al., BMC Genomics 2008, 9:5). The RNA was incubated at 95°C prior to cDNA synthesis to avoid problems caused by a possible secondary structure of the RNA. After synthesis the RNA strand was digested using RNAse H before double stranded DNA was made using a combination of the 2^{nd} strand buffer components of the Universal RiboClone® cDNA Synthesis System (Promega) and Exo-Klenow polymerase and including additional supply of the random primers. A single primer PCR reaction using primer against the random primer fixed overhang (FR20RV 5' GCC GGA GCT CTG CAG ATA TC 3'; Djikeng et al., BMC Genomics 2008, 9:5) and standard *Taq* polymerase (Invitrogen) was performed (initial denature of cDNA 95°C for 5 minutes, 40 cycles of 94°C for 30 seconds, primer annealing 60°C for 15 seconds and synthesis 72°C for 1 minute and then final synthesis 72°C for 10 minutes), and the resulting products were separated by 1% agarose gel electrophoresis. PCR products in the size range 500 - 1000 bp were excised from the gel in one piece, purified and cloned using the TOPO-TA cloning system (Invitrogen). Approximately 300 clones were sequenced and subjected to analysis.

Sequences with no significant virus host cell/tissue related hits after NCBI Blastn or BlastX analysis were subjected to analysis in the ContigExpress program of Vector NTI software, resulting in identification of two different sequences without overlap. One sequence included two contigs (contig 1 = 2,4 kB, contig 2 = 4,0 kB with an initial BlastX hit of RNA polymerase of several totivirus (best hit E=1e-04 over appr. 430bp).

The smallest sequence contig 1 (2,4kb) showed no homology to other known sequences, while the largest sequence showed weak homology with the RNA polymerase from a totivirus in a 870bp region (Expect = 7e-15, amino acid identities = 81/312 (25%), Positives = 138/312 (44%), Gaps = 32/312 (10%). The assembly of both sequences showed various numbers of individual clone sequences in different regions resulting in low quality overlap in some regions. When analyzing the sequences by PCR, it became evident that some parts of the sequences are difficult to amplify by PCR, indicating strong secondary structures in these regions. Especially PCR in the region between the end part of ORF 1 and the first part of ORF2 is predicted to contain strong secondary structures since PCR in this region is very inefficient and difficult to perform (approximately nucleotide 2800 - 3200). [Without being bound by any specific theory, it is believed that this may partly explain the difficulties experienced with the isolation and characterization of the CMS virus genome].

A sequence joining the two sequence parts was identified by PCR using primers. Two primers, PMCV-bridge F (5'-AGCAGGAGCAACAGCACAC-3') and PMCV-bridge R (5'-TACCCGCCAAATGGTACTTC-3'), were designed to span the region between the two Contigs, resulting in a product of 96 bp. By manually going through the unassigned sequences obtained, a sequence 3' of contig 2 was identified. The 5' and 3' ends of the genome were identified using 5' and 3' RACE.

Finally, a genome of 6688 bp was assembled, containing three open reading frames. This is the first time a putative member of the *Totiviridae* is identified in a vertebrate. The finding of reading frame 3 (SEQ ID No. 6) was also very surprising. No other known *Totiviridae* members have this open reading frame.

### Example 2

### Expression of recombinant protein in a prokaryot expression system.

### ORF1

Gene sequences from open reading frame 1 was made synthetically as an E.coli-optimised sequence and cloned into a pET vector system using standard methods. A suitable E.coli host cell was transformed, and recombinant protein expressed and purified as disclosed in details below.

ORF1 and a partial ORF1 consisting of the first 852 nucleotides (ORF1 (1-852)) in the open reading frame was cloned into the vector pET14b (Novagen) using standard techniques. The plasmid was transformed into OneShot ® BL21(DE3)pLysS Chemically competent *E.coli* from Invitrogen (cat no C606003). For preparation of a pre-culture, 2 colonies of each transformants were picked and grown in 3ml LB + antibiotics ((Ampenicillin (50mg/ml), Chloramphenicol (34mg/ml)).

After growth for about 7,5hours, 1ml of each pre-cultures were transferred to 50ml pre-warmed medium containing antibiotics.

The cultures were induced by the addition of IPTG (0,5mM) after about growth for 2 hours at an OD between 0.7 and 1 and the temperature was lowered to 16 °C. The cultures were grown over night at this temperature.

| **Construct** | **OD induction** | **OD harvest** |
|---|---|---|
| **ORF1(1-852)** | **0,8** | **3,09** |
| **ORF1(1-852)** | **0,78** | **3,18** |
| **ORF1** | **0,7** | **4,2** |
| **ORF1** | **0,76** | **4,2** |

The cultures were harvested 15h after induction and the bacteria were pelleted at 3300xg for 20min. The supernatants were discarded and the pellets stored at -20 °C until purification of inclusion bodies. The isolation of inclusion bodies was done using protein extraction reagents B-PER® (Pierce) according to the manufacturer's instructions.

The isolated proteins separated by gel electrophoresis using the Phast gel system according to the manufacturer's instructions (GE Healthcare Life Sciences) are shown in figure 9A.

### B. ORF3

An expression vector for ORF3 was constructed using a vector system with a Pm/xylS induction system **(**Blatny, J. M., Brautaset, T., Winther-Larsen, H. C., Haugan, K., og Valla, S. 1997a. Construction and use of a versatile set broad-host-range cloning and expression vectors based on the RK2 replicon. Appl. Environ. Microbiol. 63:370-379, Blatny, J. M., Brautaset, T., Winther-Larsen, H. C., Karunakaran, P., og Valla, S. 1997b. Improved broad-host-range RK2 vectors for high and low regulated gene expression levels in gram-negative bacteria. Plasmid 38:35-51).

The vector had the cop 271 version of the replication protein TrfA, giving a copy number of 15-30 in *E .coli*, and a c-myc and 6xHis tag fused to the 3' end of ORF3.

The plasmid was transformed into *E. coli* RV308 and fermented according to Sletta et al 2007 **(**Sletta, H., Tøndervik, A., Hakvåg, S., Aune, T. E. V., Nedal, A., Aune, R., Evensen, G., Valla, S. og Brautaset, T. 2007. The presence of N-terminal secretion signal sequences leads to strong stimulation of the total expression levels of three tested medically important proteins during high-cell-density cultivations of Escherichia coli. Appl. Environ. Microbiol. 73:906-912.)

Inclusion bodies were isolated from the fermentate using protein extraction kit B-PER® (Pierce) according to the manufacturer's instructions. The isolated proteins separated by gel electrophoresis using the Phast gel system according to the manufacturers instructions (GE Healthcare Life Sciences) are shown in figure 9B.

### Example 3

### Expression of recombinant protein in a eukaryotic expression system.

Gene sequences from open reading frame 1 and 3 was cloned into a vector system using standard methods. A suitable eukaryotic host cell was transformed, and recombinant protein expressed and then purified as described in details below.

A partial ORF1 consisting of the first 852 amino acids of the open reading frame and all of ORF3 was cloned into the vector pcDNA4 (Invitrogen) using standard techniques. HeLa cells grown on coverslips were transfected using FuGENE® (Promega) according to the manufacturer's instructions. To determine whether the open reading frames were expressed, transfected cells were fixed in 3% paraformaldehyde for 15 minutes, washed in PBS and quenched with 50 mM NH4Cl for 15 min. washed in PBS and incubated with primary anti-His antibody (Antibody commercially available from Qiagen, 1:100) for 2 hrs at room temperature. After washing with PBS, samples were incubated with secondary Ab commercially available from Jackson ImmunoResearch Laboratories, Inc. for 45 min (Goat a-mouse IgG-CY3 1:500 and Donkey a-mouse IgG-CY3 1:500, respectively). After washing with PBS, the coverslips were dipped in ddH₂O before mounting in a high quality polyvinyl alcohol anti-fade medium Mowiol® (Polysciences, Inc.) containing 2ug/ml Hoechst for staining of the nucleus.

No structures were seen one day after transfection. After two days of transfection ORF1(1-852)-transfected cells showed rather big structures containing the expressed His-tagged protein. Fewer cells transfected with ORF3 showed the same structures. The negative control cells showed no specific staining. This shows that the ORF1 and ORF3 proteins can be expressed in a eukaryotic expression system.

### Example 4

### Immunogenicity of recombinant ORF 1 proteins

Rabbits were immunized with recombinant ORF 1 produced as described above in example 2 using standard methods. Rabbits were injected with 60-100mg protein, and boosted three consecutive times. Serum was harvested after 10 weeks, and tested in Western blotting.

Heart homogenate from Atlantic salmon diagnosed with CMS and heart homogenate from healthy fish was subjected to PAGE and blotted to a PVDF (polyvinylidene fluoride) membrane using a PhastGel® apparatus (GE Healthcare). The membrane was incubated with antiserum (1:250) for 120 minutes, washed in PBS and incubated with a secondary antibody coupled to alkaline phosphatase (1:500, DAKO, Denmark) for 60 minutes. The blot was visualized with NBT-BCIP (5-bromo-4-chloro-3-indolyl-phosphate/nitro blue tetrazolium) (Promega) according to the manufacturer's instructions.

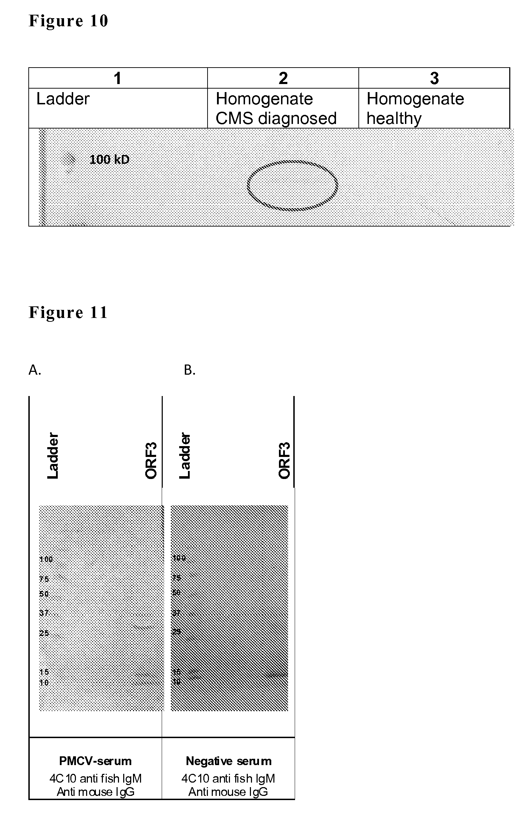

The Western blots show the presence of a specific band with molecular weight of approx 90kD in the lane with heart homogenate from Atlantic salmon diagnosed with CMS (Figure 10). This corresponds well with the estimated molecular weight of ORF1. These results show that recombinant ORF1 protein elicits an immune response producing an antiserum recognizing a protein from the CMS virus. Thus, ORF1 is a good candidate for antigen in a vaccine against CMS.

### Example 5.

### Immunogenicity of recombinant ORF 3 proteins.

Recombinant ORF 3 protein was produced as described in example 2 and subjected to PAGE and blotted to PVDF (polyvinylidene fluoride) membranes using a PhastGel® apparatus (GE Healthcare). The membranes were blocked for 2 hours at 37oC in TBS-0,05%tween (TTBS) containing 5% skimmed milk powder.

Fish serum from either Atlantic salmon diagnosed with CMS or a healthy control fish was diluted to 1:2 with TTBS+5% skimmed milk powder and the membrane was incubated O/N at 4oC. The membranes were washed 3x5min in TTBS+1% skimmed milk powder before 1 hr incubation with 4C10 mouse anti-trout IgM (1:1000) in 3x5min in TTBS+1% skimmed milk powder at RT. The membranes were washed 3x5 min in TBS-tween before 1 hr incubation with Goat-anti-mouse IgG-AP (1:500) in TTBS only. The membrane was then washed 3x5 min in TTBS before incubation at RT.for 5 minutes in NBT/BCIP substrate buffer before NBT/BCIP (Promega) was added to visualize the proteins.

The serum from the Atlantic salmon diagnosed with CMS recognised the recombinant ORF3 proteins, while the serum from the healthy fish did not (Figure 11). This shows that the recombinant ORF 3 protein has common epitopes with the virus causing CMS. Thus, ORF3 is a good candidate for antigen in a vaccine against CMS.

### Example 6

### DNA vaccine protecting against CMS

Open reading frame 1(nucleotide 1-852) and 3 were cloned into a suitable DNA vaccine vector, pcDNA4 (Invitrogen) using standard techniques. The resulting vector was injected intramuscularly into Atlantic salmon proven to be free of CMS virus in a dose of 100 µg/fish. After an immunization period of 6 weeks at 12 degrees Celsius, the fish were i.p. challenged with salmon cardiomyopathy virus. A group injected with PBS instead of vaccine was included as a control. Tissue samples from challenged vaccinated and unvaccinated fish were collected for analysis by Real Time RT-PCR to assess the viral load in the kidney three weeks after challenge. The samples were kept in RNAlater® (Ambion) until analysis. RNA was extracted using the RNeasy kit® (Quiagen). All RNA samples were diluted to 50 ng/µl prior to cDNA synthesis. For cDNA synthesis and Real Time PCR, the SuperScript^{®} III Platinum^{®} Two-Step qRT-PCR Kit with SYBR^{®} Green was used. For the Real Time PCR, the primers PMCV ORF2-3F/ORF2-3R (ORF2-3F= (5'-GGAAGCAGAAGTGGTGGAGCGT-3') and ORF2-3R= (5'-CCGGTTTTGCGCCCTTCGTC-3') were used, and the cycling conditions were: 50°C - 2 min, 95°C - 2 min, (95°C - 15 sek, 60°C - 1min) x 45 cycles.

Since the highest viral load is found in the kidney throughout the CMS virus infection, this was used as a measure for vaccine efficacy. The viral load in the kidney of the infected fish and control fish reported as cycle threshold (Schmitgen TD, LIvak KJ, Nat. Protoc. 2008; 3(6): 1101-8 "Analyzing real-time PCR data by the comparative C(T) method"), Ct were as follows:

| | **Ct average** |
|---|---|
| **ORF1(1-852)** | 27,33 |
| **ORF3** | 26,27 |
| **PBS control** | 24,62 |

A Ct value difference of 3 represents a 100 fold difference in viral load. The average Ct value from analysis of kidney tissue for the control group was 24,62. Both the group vaccinated with ORF1 (1-852) and ORF3 had higher average Ct values than the control. A higher Ct value means that the viral load was lower in the vaccinated groups than in the control group. This result shows that the ORF1(1-852) and ORF3 DNA vaccine constructs were effectively lowering the viral load in the fish and thus has a potential as DNA vaccines against CMS.

### Example 7

### PCR diagnostics

The following primers have been tested and found suitable for PCR diagnostics for the CMS virus

| **PRIMER** | **FORWARD (5' - 3')** | **REVERSE (5' - 3')** | **PROD. LENGTH** |
|---|---|---|---|
| PMCV | ACACCAGGTGACCGAAAAG | TCCAGTGCCTTGATGTCTG (SEQ ID NO. 8) | 237 bp |
| A01 | ACATGGTGCGAGGTAACGAC | CATCTCCATCCGCTAAGTACG (SEQ ID NO. 9) | 124 bp |
| B04 | AGTTCCTGCCCGTAGATGG | TGCCTGTCGTTGAGTTTAGC (SEQ ID NO. 10) | 531 bp |
| F02 | GGCGAGAATGGTGTTTGTG | CCCGAATGAAGCAAGATGG (SEQ ID NO. 11) | 269 bp |
| A09 | GAAAGCCCAGACTCAGGATG | CCTGGTCTCACTCCCAAGAG (SEQ ID NO. 17) | 242 bp |
| ORF2-3 | GGAAGCAGAAGTGGTGGAGC GT | CCGGTTTTGCGCCCTTCGTC (SEQ ID NO. 21) | 107 bp |

It is also possible to design Real Time PCR methods using primers and probes, for instance

| **PRIMER** | **FORWARD (5' - 3')** | **REVERSE (5' - 3')** | **PROBE** |
|---|---|---|---|
| PMCV-F2 | AGGGAACAGGAGGAAGCAGAA | CGTAATCCGACATCATTTTGT GA (SEQ ID NO. 19) | TGGTGGAGCG TTCAA |

The 5'half of the virus genome is much richer in GC content than the 3' part. We predict that PCR in the 3' part of the genome is more efficient than in the 5' end. There is also a part in the middle of the genome in the region between and partially overlapping with ORF1 and ORF 2 forming a pseudoknot, thus making PCR in this region difficult.

### Example 8

### Clinical CMS correlates with presence of PMCV in heart tissue.

To examine whether clinical CMS in farmed fish coincides with the presence of PMCV in heart tissue, samples were collected from three fish farms. Two of these (1 and 2) had a CMS diagnosis, while site three had no prior history of CMS. Real-time RT-PCR on field samples was run on the MXpro 3000P (Stratagene) with all reactions performed in triplicate using the following primers:
ORF2-3F (5'-GGAAGCAGAAGTGGTGGAGCGT-3') and
ORF2-3R (5'-CCGGTTTTGCGCCCTTCGTC-3').

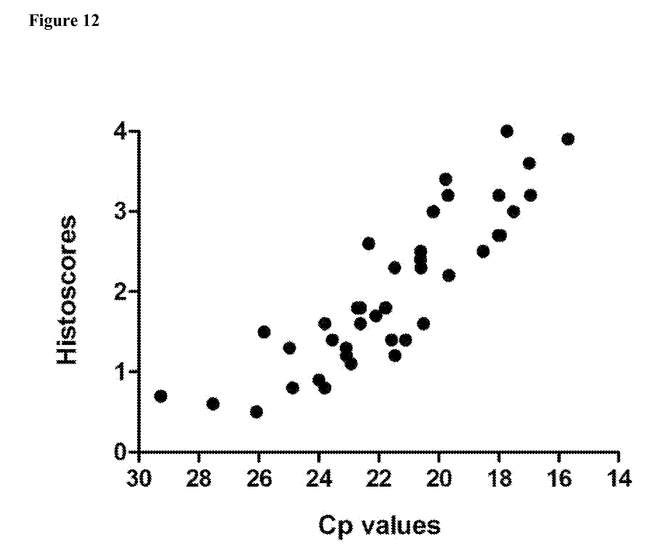

Fish collected from Sample 1 (at slaughter) were found with histomorphological changes typical of CMS with scores ranging from 0.6 to 4 (covering the entire scale; Fig. 12). The relationship between histoscores and Cp values as assessed by real-time RT-PCR shows that there is a clear tendency that higher virus amount being found in heart tissue with high histoscores (r2=0.76). As it has been suggested that the biggest fish die most frequently from CMS the Cp values were also plotted against the condition factor (weightx100/length) of the fish without any correlation being found (r2=0.08).

For Sample 2 (clinical CMS), 10 out of 10 fish were positive by real-time RT-PCR and all showed typical histopathological changes of CMS.

For Sample 3 (previous history of HSMI), no fish were found positive for PMCV by real-time RT-PCR while all were found positive by real-time RT-PCR for PRV. None of the fish had changes typical of CMS.

These findings show that clinical cases of CMS correlate with presence of PMCV in heart tissue and further substantiate that PMCV is the causative agent of CMS.

## Claims

1. An isolated nucleic acid sequence originating from CMS virus having a sequence selected from the group consisting of SEQ ID No. 1, SEQ ID No. 2, SEQ ID No. 4 and SEQ ID No. 6, and variants thereof being at least 70 % identical over the entire sequence with any of the sequences SEQ ID No. 2, SEQ ID No. 4 and SEQ ID No. 6.

2. An isolated nucleic acid sequence according to claim 1, wherein the sequence is at least 80%, preferably 90 %, more preferably 95% identical over the entire sequence with the nucleic acid selected from the group consisting of SEQ ID No. 2, SEQ ID No. 4 and SEQ ID No. 6.

3. A vector comprising a nucleic acid sequence according to the claims 1-2.

4. A vector according to claim 3 comprising a sequence selected from the group consisting of SEQ ID No. 2 and SEQ ID No. 6, and variants thereof being at least 70 % identical over the entire sequence with any of the sequences SEQ ID No. 2, and SEQ ID No. 6, wherein said isolated nucleic acid sequence is operatively linked to control sequences directing the expression of said sequences.

5. Vector according to claim 3-4, wherein the vector is a pET vector.

6. A host cell comprising a vector according to any of the claims 4-5.

7. A DNA vaccine comprising a nucleic acid sequence selected from the group consisting of SEQ ID No. 2 and SEQ ID No. 6, and variants thereof being at least 70 % identical over the entire sequence with any of the sequences SEQ ID No. 2, and SEQ ID No. 6.

8. A recombinant protein encoded by a nucleic acid sequence selected from the group consisting of SEQ ID No. 2 and SEQ ID No. 6, and variants thereof being at least 70 % identical over the entire sequence with any of the sequences SEQ ID No. 2, and SEQ ID No. 6.

9. A recombinant protein according to claim 8 having an amino acid sequence selected from the group consisting of SEQ ID No. 3 and SEQ ID No. 7, or a variant thereof being at least 70 % identical over the entire sequence with any of the sequences SEQ ID No. 3, and SEQ ID No. 7.

10. A recombinant vaccine comprising at least one recombinant protein according to claim 8-9.

11. An antibody specifically recognising and specifically binding to a recombinant protein according to claims 8-9.

12. An antibody according to claim 11, wherein said antibody is a monoclonal antibody.

13. An antibody according to claim 11, wherein said antibody is polyclonal.

14. An immunoassay for detection of CMS virus specific antibody against CMS virus in a biological sample, **characterized by** comprising the following steps:
a) contacting at least one of the recombinant proteins according to any of the claims 8-9 and a biological sample suspected to comprise antibodies against a CMS virus; and
(b) determining whether the at least one recombinant protein binds to a CMS virus specific antibody present in the biological sample wherein said binding represents an indication that the animal has been in contact with CMS virus.

15. An immunoassay for detection of CMS virus in a biological sample, **characterized by** comprising the following steps:
(a) contacting one or more antibodies that specifically recognise and bind to a recombinant protein according to claim 8-9 and a biological sample suspected to comprise CMS virus; and
(b) determining whether the one or more antibody binds to CMS virus present in the biological sample wherein said binding represents an indication that the sample contains a CMS virus.

16. A method according to claim 15, wherein the antibody is a monoclonal antibody.

17. A primer comprising a sequence of at least 15 nucleotides, wherein said primer hybridizes specifically to a nucleic acid sequence originating from CMS virus, having a sequence selected from the group consisting of SEQ ID No. 1, SEQ ID No. 2, SEQ ID No. 4 and SEQ ID No. 6, and variants thereof being at least 70 % identical over the entire sequence with any of the sequences SEQ ID No. 2, SEQ ID No. 4 and SEQ ID No. 6.

18. A primer according to claim 17, wherein said primer is at least 20 nucleotides.

19. A primer according to claim 17, wherein said primer is selected from the group consisting of SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17 SEQ ID No. 18, SEQ ID No. 19, SEQ ID No. 20 and SEQ ID No. 21.

20. A method for detection of CMS virus in a biological sample **characterised by** comprising the following steps:
a) preparing a biological sample comprising nucleic acid sequences isolated from a biological sample suspected to comprise CMS virus for a reverse transcription reaction;
b) subjecting the mixture of a) to a polymerase chain reaction with a primer pair selected from primers according to claims 17-19; and
c) determining whether the binding of the primers to nucleic acid sequences in the sample and amplification of the sequence between them have occurred indicating the presence of CMS virus in the sample tested.

21. A method according to claim 20, wherein said primer of the primer pair is selected from a group consisting of SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17 SEQ ID No. 18, SEQ ID No. 19, SEQ ID No. 20 and SEQ ID No. 21.

22. A diagnostic kit comprising at least one primer sequence according to any of the claims 17-19.

23. The use of the nucleic acid sequences according to any of the claims 1-2 for the preparation of a DNA vaccine, a recombinant vaccine or a live recombinant microorganism.

## Patentansprüche

1. Isolierte Nucleinsäuresequenz, die von CMS-Virus stammt, mit einer Sequenz ausgewählt aus der Gruppe bestehend aus SEQ ID No. 1, SEQ ID No. 2, SEQ ID No. 4 und SEQ ID No. 6 und Varianten davon, die über die gesamte Sequenz zu mindestens 70% identisch mit irgendeiner der Sequenzen SEQ ID No. 2, SEQ ID No. 4 und SEQ ID No. 6 sind.

2. Isolierte Nucleinsäuresequenz nach Anspruch 1, wobei die Sequenz über die gesamte Sequenz zu mindestens 80%, bevorzugt 90%, bevorzugter 95% identisch mit der Nucleinsäure ausgewählt aus der Gruppe bestehend aus SEQ ID No. 2, SEQ ID No. 4 und SEQ ID No. 6 ist.

3. Vektor, umfassend eine Nucleinsäuresequenz nach den Ansprüchen 1-2.

4. Vektor nach Anspruch 3, umfassend eine Sequenz ausgewählt aus der Gruppe bestehend aus SEQ ID No. 2 und SEQ ID No. 6 und Varianten davon, die über die gesamte Sequenz zu mindestens 70% identisch mit irgendeiner der Sequenzen SEQ ID No. 2 und SEQ ID No. 6 sind, wobei die isolierte Nucleinsäuresequenz operativ verknüpft ist mit Kontrollsequenzen, die die Expression der Sequenzen steuern.

5. Vektor nach Anspruch 3-4, wobei der Vektor ein pET-Vektor ist.

6. Wirtszelle, umfassend einen Vektor nach irgendeinem der Ansprüche 4-5.

7. DNA-Vakzine, umfassend eine Nucleinsäuresequenz ausgewählt aus der Gruppe bestehend aus SEQ ID No. 2 und SEQ ID No. 6 und Varianten davon, die über die gesamte Sequenz zu mindestens 70% identisch mit irgendeiner der Sequenzen SEQ ID No. 2 und SEQ ID No. 6 sind.

8. Rekombinantes Protein, kodiert durch eine Nucleinsäuresequenz ausgewählt aus der Gruppe bestehend aus SEQ ID No. 2 und SEQ ID No. 6 und Varianten davon, die über die gesamte Sequenz zu mindestens 70% identisch mit irgendeiner der Sequenzen SEQ ID No. 2 und SEQ ID No. 6 sind.

9. Rekombinantes Protein nach Anspruch 8 mit einer Aminosäuresequenz ausgewählt aus der Gruppe bestehend aus SEQ ID No. 3 und SEQ ID No. 7 oder einer Variante davon, die über die gesamte Sequenz zu mindestens 70% identisch mit irgendeiner der Sequenzen SEQ ID No. 3 und SEQ ID No. 7 ist.

10. Rekombinante Vakzine, umfassend mindestens ein rekombinantes Protein nach Anspruch 8-9.

11. Antikörper, der ein rekombinantes Protein nach den Ansprüchen 8-9 spezifisch erkennt und spezifisch daran bindet.

12. Antikörper nach Anspruch 11, wobei der Antikörper ein monoklonaler Antikörper ist.

13. Antikörper nach Anspruch 11, wobei der Antikörper polyklonal ist.

14. Immunoassay für den Nachweis von CMS-Virus-spezifischem Antikörper gegen CMS-Virus in einer biologischen Probe, **dadurch gekennzeichnet, dass** es folgende Schritte umfasst:
a) Inkontaktbringen von mindestens einem der rekombinanten Proteine nach irgendeinem der Ansprüche 8-9 mit einer biologischen Probe, die unter dem Verdacht steht, Antikörper gegen ein CMS-Virus zu umfassen; und
(b) Bestimmen, ob das mindestens eine rekombinante Protein an einen CMS Virus-spezifischen Antikörper, der in der biologischen Probe vorhanden ist, bindet, wobei diese Bindung einen Hinweis darstellt, dass das Tier in Kontakt mit CMS-Virus gewesen war.

15. Immunoassay für den Nachweis von CMS-Virus in einer biologischen Probe, **dadurch gekennzeichnet, dass** es folgende Schritte umfasst:
(a) Inkontaktbringen von einem oder mehreren Antikörpern, die ein rekombinantes Protein nach Anspruch 8-9 spezifisch erkennen und daran binden, mit einer biologischen Probe, die unter dem Verdacht steht, CMS-Virus zu umfassen; und
(b) Bestimmen, ob das eine oder die mehreren Antikörper an in der biologischen Probe vorhandenem CMS-Virus binden, wobei diese Bindung einen Hinweis darstellt, dass die Probe ein CMS-Virus enthält.

16. Verfahren nach Anspruch 15, wobei der Antikörper ein monoklonaler Antikörper ist.

17. Primer, umfassend eine Sequenz von mindestens 15 Nucleotiden, wobei der Primer spezifisch an eine von CMS-Virus stammende Nucleinsäuresequenz mit einer Sequenz ausgewählt aus der Gruppe bestehend aus SEQ ID No. 1, SEQ ID No. 2, SEQ ID No. 4 und SEQ ID No. 6 und Varianten davon, die über die gesamte Sequenz zu mindestens 70% identisch mit irgendeiner der Sequenzen SEQ ID No. 2, SEQ ID No. 4 und SEQ ID No. 6 sind, hybridisiert.

18. Primer nach Anspruch 17, wobei der Primer mindestens 20 Nucleotide ist.

19. Primer nach Anspruch 17, wobei der Primer ausgewählt ist aus der Gruppe bestehend aus SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20 und SEQ ID No. 21.

20. Verfahren für den Nachweis von CMS-Virus in einer biologischen Probe,
**dadurch gekennzeichnet, dass** es folgende Schritte umfasst:
a) Herstellen einer biologischen Probe, umfassend Nucleinsäuresequenzen isoliert von einer biologischen Probe, die im Verdacht steht, CMS-Virus zu umfassen, für eine reverse Transkriptionsreaktion;
b) Unterwerfen der Mischung von a) einer Polymerase-Kettenreaktion mit einem Primerpaar ausgewählt aus Primern nach den Ansprüchen 17-19; und
c) Bestimmen, ob die Bindung der Primer an Nucleinsäuresequenzen in der Probe und die Amplifikation der Sequenz zwischen ihnen stattgefunden haben, was auf die Anwesenheit von CMS-Virus in der untersuchten Probe hinweist.

21. Verfahren nach Anspruch 20, wobei der Primer des Primerpaars ausgewählt ist aus einer Gruppe bestehend aus SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20 und SEQ ID No. 21.

22. Diagnosekit, umfassend mindestens eine Primersequenz nach irgendeinem der Ansprüche 17-19.

23. Verwendung der Nucleinsäuresequenzen nach irgendeinem der Ansprüche 1-2 zur Herstellung einer DNA-Vakzine, einer rekombinanten Vakzine oder eines lebenden rekombinanten Mikroorganismus.

## Revendications

1. Séquence d'acide nucléique isolée provenant d'un virus CMS ayant une séquence choisie dans le groupe constitué par SEQ ID NO.: 1, SEQ ID NO. 2, SEQ ID NO. 4 et SEQ ID NO. 6, et les variants de celles-ci qui sont identiques à au moins 70% sur la séquence entière avec l'une quelconque des séquences SEQ ID NO. 2, SEQ ID NO. 4 et SEQ ID NO. 6.

2. Séquence d'acide nucléique isolée selon la revendication 1, laquelle séquence est identique à au moins 80%, de préférence 90%, de préférence encore 95% sur la séquence entière avec l'acide nucléique choisi dans le groupe constitué par SEQ ID NO. 2, SEQ ID NO. 4 et SEQ ID NO. 6.

3. Vecteur comprenant une séquence d'acide nucléique selon les revendications 1-2.

4. Vecteur selon la revendication 3, comprenant une séquence choisie dans le groupe constitué par SEQ ID
NO. 2 et SEQ ID NO. 6, et les variants de celles-ci qui sont identiques à au moins 70% sur la séquence entière avec l'une quelconque des séquences SEQ ID NO. 2 et SEQ ID NO. 6, dans lequel ladite séquence d'acide nucléique isolée est liée de manière fonctionnelle à des séquences de contrôle dirigeant l'expression desdites séquences.

5. Vecteur selon les revendications 3-4, lequel vecteur est un vecteur pET.

6. Cellule hôte comprenant un vecteur selon l'une quelconque des revendications 4-5.

7. Vaccin à ADN comprenant une séquence d'acide nucléique choisie dans le groupe constitué par SEQ ID NO. 2 et SEQ ID NO. 6, et les variants de celles-ci qui sont identiques à au moins 70% sur la séquence entière avec l'une quelconque des séquences SEQ ID NO. 2 et SEQ ID NO. 6.

8. Protéine recombinante codée par une séquence d'acide nucléique choisie dans le groupe constitué par SEQ ID NO. 2 et SEQ ID NO. 6, et les variants de celles-ci qui sont identiques à au moins 70% sur la séquence entière avec l'une quelconque des séquences SEQ ID NO. 2 et SEQ ID NO. 6.

9. Protéine recombinante selon la revendication 8 ayant une séquence d'acides aminés choisie dans le groupe constitué par SEQ ID NO. 3 et SEQ ID NO. 7, ou un variant de celles-ci qui est identique à au moins 70% sur la séquence entière avec l'une quelconque des séquences SEQ ID NO. 3 et SEQ ID NO. 7.

10. Vaccin recombinant comprenant au moins une protéine recombinante selon les revendications 8-9.

11. Anticorps reconnaissant spécifiquement et se liant spécifiquement à une protéine recombinante selon les revendications 8-9.

12. Anticorps selon la revendication 11, ledit anticorps étant un anticorps monoclonal.

13. Anticorps selon la revendication 11, ledit anticorps étant polyclonal.

14. Immunoessai pour la détection d'anticorps, spécifiques du virus CMS, dirigés contre un virus CMS dans un échantillon biologique, **caractérisé en ce qu'**il comprend les étapes suivantes :
a) mise en contact d'au moins l'une des protéines recombinantes selon l'une quelconque des revendications 8-9 et d'un échantillon biologique soupçonné de comprendre des anticorps contre un virus CMS ; et
(b) détermination si ladite au moins une protéine recombinante se lie à un anticorps spécifique à un virus CMS présent dans l'échantillon biologique, dans laquelle ladite liaison représente une indication que l'animal a été en contact avec un virus CMS.

15. Immunoessai pour la détection de virus CMS dans un échantillon biologique, **caractérisé en ce qu'**il comprend les étapes suivantes :
(a) mise en contact d'un ou plusieurs anticorps qui reconnaissent et se lient spécifiquement à une protéine recombinante selon les revendications 8-9 et d'un échantillon biologique soupçonné de comprendre un virus CMS ; et
(b) détermination si lesdits un ou plusieurs anticorps se lient à un virus CMS présent dans l'échantillon biologique, dans laquelle ladite liaison représente une indication que l'échantillon contient un virus CMS.

16. Procédé selon la revendication 15, dans lequel l'anticorps est un anticorps monoclonal.

17. Amorce comprenant une séquence d'au moins 15 nucléotides, ladite amorce s'hybridant spécifiquement à une séquence d'acide nucléique provenant de virus CMS, ayant une séquence choisie dans le groupe constitué par SEQ ID NO. 1, SEQ ID NO. 2, SEQ ID NO. 4 et SEQ ID NO. 6, et les variants de celles-ci qui sont identiques à au moins 70% sur la séquence entière avec l'une quelconque des séquences SEQ ID NO. 2, SEQ ID NO. 4 et SEQ ID NO. 6.

18. Amorce selon la revendication 17, ladite amorce étant d'au moins 20 nucléotides.

19. Amorce selon la revendication 17, ladite amorce étant choisie dans le groupe constitué par SEQ ID No. 8, SEQ ID NO. 9, SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 18, SEQ ID NO. 19, SEQ ID NO. 20 et SEQ ID NO. 21.

20. Procédé pour détecter un virus CMS dans un échantillon biologique, **caractérisé en ce qu'**il comprend les étapes suivantes :
a) préparation d'un échantillon biologique comprenant des séquences d'acides nucléiques isolées à partir d'un échantillon biologique soupçonné de comprendre un virus CMS pour une réaction de transcription inverse ;
b) soumission du mélange de a) à une réaction en chaîne de polymérase avec une paire d'amorces choisies parmi les amorces selon les revendications 17 à 19 ; et
c) détermination si la liaison des amorces à des séquences d'acides nucléiques dans l'échantillon et une amplification de la séquence entre elles se sont produites, indiquant la présence de virus CMS dans l'échantillon testé.

21. Procédé selon la revendication 20, dans lequel ladite amorce de la paire d'amorces est choisie dans un groupe constitué par SEQ ID NO. 8, SEQ ID NO. 9, SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 18, SEQ ID NO. 19, SEQ ID NO. 20 et SEQ ID NO. 21.

22. Kit de diagnostic comprenant au moins une séquence d'amorce selon l'une quelconque des revendications 17 à 19.

23. Utilisation des séquences d'acides nucléiques selon l'une quelconque des revendications 1-2 pour la préparation d'un vaccin à ADN, d'un vaccin recombinant ou d'un micro-organisme recombinant vivant.
